# EUROPEAN PATENT APPLICATION

(11) **EP 4 464 285 A1**
(43) Date of publication of application: **20.11.2024**
(21) Application number: 23173394.0
(22) Date of filing: 15.05.2023
(51) Int. Cl.: A61F 2/954, A61M 25/10

(54) **DEVICE AND METHODS FOR TREATING BIFURCATED BLOOD VESSELS**

(71) Applicant: Bentley InnoMed GmbH, 72379 Hechingen (DE)
(72) Inventor: BREGULLA, Rainer, 72336 Balingen (DE)
(74) Representative: Hoefer & Partner Patentanwälte mbB

(57) **Abstract**

The invention relates to a balloon catheter for delivering a bifurcated stent into a bifurcated blood vessel, the balloon catheter comprising: a hub; a shaft; and a single balloon connected to the shaft; an inflation port at the hub in fluid communication with the single balloon; wherein the single balloon comprises a first distal portion, a second central portion and a third proximal portion, wherein in its delivery configuration the first distal portion of the balloon is bent down to the third proximal portion of the balloon such that the first distal portion and the third proximal portion of the balloon are arranged adjacent to each other.

## Description

### Field

The invention relates to medical devices and methods for treating lesions in a bifurcated blood vessel. The invention is further directed to a system for treating aorto-iliac occlusive disease (AIOD) in a retrograde approach.

### Background

Aorto-iliac occlusive disease (AIOD) is a narrowing or stenosis of the blood vessels involving the abdominal aorta as it transitions into the two iliac arteries. This narrowing or blockage is typically caused by a buildup of plaque in the aorta, the aortic bifurcation and into the iliac arteries. As a result of the plaque, diameters of the flow lumens in the iliac arteries are reduced, thereby restricting blood flow to the patient's legs and organs within the pelvis.

Endoluminal procedures for treatment of AIOD usually includes placement of a bare or covered stent within the bifurcation. The stent can be self-expanding or balloon expandable. Two treatment types are generally used for treating AIOD involving the aorto-iliac bifurcation, including kissing stents and covered endovascular reconstruction of aortic bifurcation (CERAB). Kissing stents is a procedure where two stents are seated at the aortic bifurcation and cross each other above the aortic bifurcation. Similarly, covered endovascular reconstruction is a procedure where a main stent graft body is implanted into the aorta above the aortic bifurcation and separate stent graft branches for each of the iliac arteries are implanted to cross each other within the main stent graft body above the aortic bifurcation.

Both techniques include simultaneous placement of two parallel or "kissing" stents across the bifurcation as well as the placement of a stent or stent graft in the aorta.

A common shortcoming of these two techniques is the disruption of the natural blood flow through the bifurcation that can lead to thrombosis, hemolysis, emboli and restenosis. Another shortcoming is the need for accurate simultaneous placement of the two kissing stents and the high technical skill set required to perform this procedure. Also, the two kissing stents make re-intervention procedures using a retrograde approach (up-and-over technique) very challenging. Further yet, another shortcoming is that using conventional balloons for dilatation or re-dilatation of the main stent in the aorta prevents placement of the stent in direct proximity to the bifurcation as the balloon overhang which is necessarily relatively long for balloons with a diameter suiting the aorta, forces the physician to keep a certain distance to the bifurcation to not risk an over-dilatation of the vessel in this area. With the balloon overhang being symmetrically on both ends, this shortcoming exists for both approaches retrograde or antegrade, where a retrograde approach is widely preferred due to less complications during the procedure.

There is an obvious need for medical devices and methods for the treatment of AIOD involving the aorto-iliac bifurcation that overcome the above listed shortcomings of existing methods. These shortcomings are addressed by the medical device and methods for treating bifurcated blood vessels involving the aorto-iliac bifurcation described herein.

### Description

Described herein generally are medical devices and methods for the treatment of any disease involving a bifurcation in a body lumen. In some embodiments, the devices and methods are for the treatment of aorto-iliac occlusive disease (AOID). The devices and methods described herein can also be used for the treatment of an aortic aneurysm or any other disease(s) in the body involving a bifurcation in a body lumen.

For consistency, when describing the present devices the direction toward the external end of the catheter outside the body is referred to as "proximal" and the direction away from the external end of the catheter is referred to as "distal". The side on which the catheter is inserted in peripheral arteries is referred to as "ipsi-lateral", the opposite side is referred to as "contra-lateral". For example, if the catheter is inserted into an artery of the right leg, the right side of the body is referred to as ipsi-lateral and the right iliac artery is referred to as the ipsi-lateral iliac artery. The left side is referred to as contra-lateral and the left iliac artery is referred to as the contra-lateral iliac artery.

In some embodiments, a bifurcated stent comprises a main body stent, a first branch stent, and a second branch stent. In other embodiments, a bifurcated stent comprises a main body stent and one branch stent. In some embodiments, the bifurcated stent can comprise more than two branch stents. In some embodiments the bifurcated stent comprises a main body and two non-tubular stent extension to reach into the side branches of the vessel.

The bifurcated stent can be a bare metal stent. In other embodiments, the bifurcated stent can be a coated stent. In other embodiments the stent can be a covered stent, also called a stent graft.

The stents can be covered with a layer of polymer or animal tissue. Covers can be made from woven or knitted fibers, polymers, porous material, or non-porous material. Cover material can be made from polyurethane, PET (polyethylene terephthalate), non-expanded PTFE (polytetrafluoroethylene), expanded ePTFE, or ultrahigh density polyethylene (UHDPE). The cover can be connected to stent by any suitable means well known in the art, like fibers, sutures, adhesives, or by clamping the cover in between stent struts, sintering, gluing, fusing, bonding or encapsulating the stent by the membrane.

The stent can be composed of a metal, metal alloy, a polymer(s), and/or a combination thereof. The stent cover can be made from a polymer or mammalian tissue. The bifurcated stent can comprise a series of stent rings connected by longitudinal struts or via a cover.

In some embodiments, the stent can be laser cut from a metal tube or polymer tube. In other embodiments, the stent can be formed from a metal wire or a polymer wire. In some embodiments, the main body stent and the two branch stents can be welded together or connected by another mechanism, such as but not limited to, suture(s), adhesive(s), connector(s), bonding(s) and/or a combination thereof. In some embodiments, the main body stent and two branch stents are connected by a material different than the material used to compose the stents.

The bifurcated stent can comprise a series of individual stent rings connected by the cover. The bifurcated stent can comprise at least two overlapping stents wherein the proximal segment of each stent forms a respective branch and the distal segment of each stent forms the main body. In some embodiments, the bifurcated stent comprises two or more overlapping stents. In some embodiments the main body stent and the two branch stents are formed integrally, i.e. the bifurcated stent is manufactured in one piece. In other embodiments the bifurcated stent is formed from 3 individual stents which are welded together.

A catheter for delivering the bifurcated stent comprises a catheter shaft and a single balloon mounted onto a distal portion of the catheter shaft. In other embodiments, the catheter includes a catheter shaft and two balloons, a first single balloon mounted on a distal portion of the catheter shaft and a second balloon mounted on the catheter shaft at a location proximal of the first balloon.

The catheter shaft includes an inflation lumen in fluid communication with a hub at the proximal end of the shaft (external to the patient's body) and in fluid communication with the single balloon.

In another embodiment where the catheter comprises two balloons the catheter includes two inflation lumen, a first inflation lumen in fluid communication with a first hub at the proximal end of the shaft and in fluid communication with a first balloon and a second inflation lumen in fluid communication with a second hub at the proximal end of the shaft and in fluid communication with a second balloon, such that the two balloons can be inflated separately.

The catheter shaft further includes a guide wire lumen, the guidewire lumen connecting a first opening in the catheter shaft at the distal end of the catheter and a second opening at the proximal end of the catheter. The guide wire lumen hosts a preloaded guide wire. The catheter can be in an over-the-wire configuration or constructed as a rapid-exchange catheter. In another embodiment, the catheter shaft includes a fixed guidewire. When a fixed guide wire is employed the catheter does not necessarily need a guide wire lumen. Instead, the guide wire is integrated within the shaft or in another embodiment directly attached to the shaft.

The single balloon can be of any shape. In some embodiments, the balloon can be of a cylindrical shape. In other embodiments, the balloon has a first distal portion, a second central portion and a third proximal portion. Each of the portions may be of any shape. Each portion may have the same shape as the other two portions, each portion may have an individual shape or the first and third portions may have the same shape whereas the second portion has another shape. In some embodiments, the balloon comprises at least a first waist portion between the first distal portion and the second central portion and at least a second waist portion between the second central portion and the third proximal balloon portion. In the context of this application a waist portion is defined as an area of the balloon having a reduced outer diameter compared to the adjacent balloon portions flanking both ends of the waist portion. The reduction of diameter in the waist portion is at least 50% compared to the adjacent balloon portions. It is explicitly stated that the balloon wall is not attached to the catheter shaft in the area of the waist portion. That means, that the balloon has one single lumen and one single in- or deflation lumen, i.e. the balloon segments share one single lumen, namely the balloon lumen.

In some embodiments the individual balloon portions may be of rotational symmetric shape, cylindrical shape, conical shape, or spherical-like shape, or concave or convex shape, or stepped shape or any combination thereof. In case the individual balloon portions are of a stepped shape, the balloon diameter of the larger balloon portion is increased by a maximum of 50% compared to the smaller balloon portion diameter along the step. In one embodiment a proximal portion of the third proximal balloon portion has a diameter which is increased by up to 50% over a distal portion of the third proximal balloon portion. In another embodiment a distal portion of the first distal balloon portion has a diameter which is increased up to 50% over a proximal portion of the first distal balloon portion. In another embodiment. In another embodiment, both, a distal portion of the first distal balloon portion and a proximal portion of the third proximal balloon portion have a diameter which is up to50% increased over a proximal portion of the first distal balloon portion and/or over a distal portion of the third proximal balloon portion.

In some embodiments the central balloon portion may be of spherical shape. In further embodiments the central portion itself comprises one or more additional waist portions, i.e. portions of reduced outer diameter compared to the adjacent balloon portions.

The single balloon can have a first distal portion including at least a first diameter, a second central portion including at least one second diameter and a third distal segment including a third diameter. In some embodiments, the first diameter is smaller than the second diameter. In other embodiments, the third diameter is smaller than the second diameter. In some embodiments, the first diameter and the third diameter are the same. In some embodiments, the first, the second and the third diameter are the same. In further embodiments each balloon portion may be of an individual shape as for example and not limitation of rotational symmetric shape, cylindrical shape, conical shape, or spherical-like shape, or concave or convex shape, or stepped shape or any combination thereof.

The catheter can be transitioned from a delivery configuration to a retracting configuration.

In its delivery configuration the first distal portion of the single balloon is bent down to the third proximal portion of the balloon such that the first distal portion and the second proximal portion of the balloon are arranged adjacent to each other. In one embodiment the first distal portion and the second proximal portion of the balloon are arranged substantially parallel to each other. In one embodiment the first distal portion and the second proximal portion of the balloon are arranged parallel to each other. In one embodiment a proximal portion of the distal portion of the balloon and a distal portion of the proximal portion of the balloon are arranged in a parallel configuration to each other, i.e. the first distal portion and the third proximal portion of the balloon are arranged at least partially in a parallel configuration to each other. In the delivery configuration of the catheter where the first distal portion of the balloon is bent down to the third proximal portion of the balloon the middle portion of the balloon serves as bending position of the balloon. The middle portion of the balloon allows the balloon to bend by 180° without kinking or blocking the blocking the balloon lumen.

The bifurcated stent can be mounted onto a catheter comprising a balloon having 3 balloon portions by mounting a first branch stent of the bifurcated stent onto a first balloon portion, a second branch stent of the bifurcated stent onto a third balloon portion, and a main body of the bifurcated stent onto the first balloon portion and third balloon portion. The second middle portion of the balloon does not carry any stent. The middle portion serves as the bending position of the balloon.

Methods of placing a stent into the aorto-iliac bifurcation are also described herein. The methods can utilize the stents, bifurcated stents and catheters described herein.

In some embodiments, the method for placing a stent into a vessel in immediate proximity to a bifurcation in a vessel of a patient comprises: a) mounting a main body stent onto a catheter having a single balloon having a first distal portion, a second central portion and a third proximal portion; wherein the main body stent is crimped onto a proximal portion of the first distal portion and onto a distal portion of the third proximal balloon portion, wherein the first balloon portion is bent down on the third balloon portion; b) advancing the catheter with the stent mounted onto the balloon from an ipsi-lateral side vessel past the bifurcation, c) placing the stent in the main vessel of a patient in immediate proximity to a bifurcation of the vessel into the bifurcating blood vessel by partially retracting the catheter; d) expanding the main body stent into the main vessel by inflating the single balloon, e) deflating the balloon, f) retracting the catheter through the ipsi-lateral side vessel, and g) removing the catheter from the patient.

In one embodiment a method for treating a lesion in a bifurcated blood vessel including a main vessel and two branch vessels, comprises: a) mounting a first branch of a bifurcated stent onto a first balloon portion and a second branch of the bifurcated stent onto a third balloon portion, and the main body of the bifurcated stent onto both the first balloon portion and third balloon portion of a balloon catheter; b) advancing the catheter with the stent mounted onto the balloon portions from an ipsi-lateral side vessel past the bifurcation; c) placing the bifurcated stent into the bifurcation such that the main body is in direct proximity to the bifurcation by partially retracting the catheter; d) expanding the bifurcated stent into the the bifurcation by inflating the single balloon, e) deflating the balloon; f) retracting the catheter through the ipsi-lateral side vessel, and g) removing the catheter from the patient.

In some embodiments a snare might be advanced from the contra-lateral side to capture the guide wire and facilitate placement of the second stent in the contra-lateral vessel or to facilitate placement of the main stent in direct proximity to the bifurcation.

If needed, post-ballooning of the bifurcated stent can be performed to conform the main body stent and the at least two branch stents to the vessel wall of the main body of the vessel and the vessel branches, respectively. The same steps or similar steps can be used to treat various different bifurcations in the body.

### Brief Description of Drawings

Figs. 1A and B illustrate conventional placement of a stent in the main vessel of a bifurcation.
Fig. 2 illustrates a catheter device according to the present application in its delivery configuration.
Fig. 3 illustrates a catheter device according to the present application in its retracting configuration
Fig. 4 illustrates another embodiment of the catheter device described herein.
Figs. 5A and B illustrate a medical device according to the present invention.
Figs. 6A to C illustrate another embodiment of the medical device according to the present invention.
Figs. 7A-D illustrate a medical device described herein.
Figs. 8A-D illustrate another embodiment of the medical device described herein.
Figs. 9A-C illustrate another embodiment of the medical device described herein.
Figs. 10A-C illustrate another embodiment of the medical device described herein.
Figs. 11A-C illustrate another embodiment of the medical device described herein.

### Detailed Description

Reference will now be made in detail to the present preferred embodiments of the invention, examples of which are illustrated in the accompanying drawings. The method and corresponding steps of the invention will be described in conjunction with the detailed description of the intravascular stent delivery catheter device.

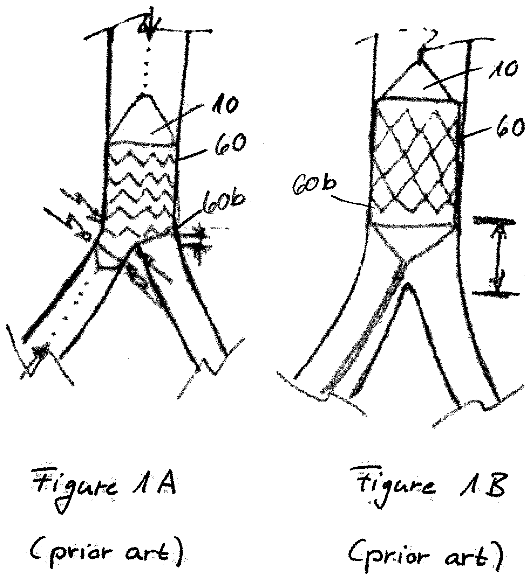

When treating aorto-iliac occlusive disease (AIOD) in a conventional way by e.g. the CERAB technique one stent or stent graft has to be placed into the aorta. In order to optimize the outcome of the stenting procedure, it is highly beneficial to place this main stent as close to the bifurcation site as possible. First, such a close placement ensures good opening of the stenosed aorta and second close placement of the aortic stent at the bifurcation site ensures that the neo bifurcation created by stenting the bifurcation is as close to the original vessel anatomy as possible. However, the balloon 10 of conventional delivery catheters come along with a relatively large balloon overhang mainly due to the relatively large size of these balloons. As can be seen in Fig. 1A, due to the balloon overhang there is a high risk of over-dilatation of the side branch vessel in case the stent 60 is placed too close to the bifurcation. On the other hand, once it is made sure that the balloon overhang does not over-dilatate the side branches, the distance between the proximal stent end 60b and the bifurcation is too far to allow for a smooth stenting of the bifurcation (see Fig 1B). This problem of the current procedure is independent of whether treatment is performed retrograde ("from below") or antegrade ("from above") as the balloon overhang on the distal end of the stent is similar to the balloon overhang on the proximal side of the balloon. Due to less side effects physicians generally prefer the retrograde approach.

According to the present invention, a catheter device is provided that overcomes the above-mentioned problems and allows for precise stenting of a bifurcated vessel employing a retrograde approach.

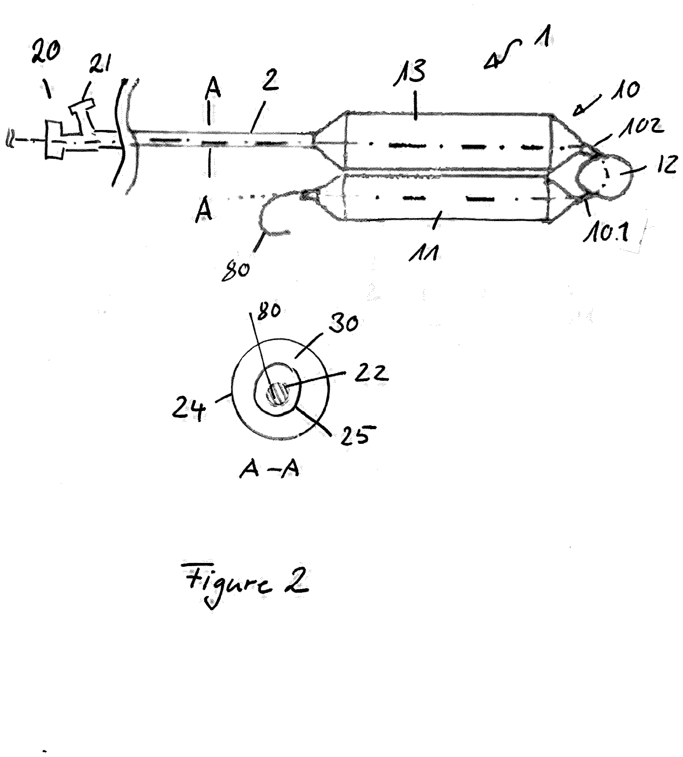

Fig. 2 illustrates a catheter device according to the present application in its delivery configuration. The balloon catheter 1 for delivering a bifurcated stent into a bifurcated blood vessel, comprises a hub 20; a shaft 2; and a single balloon 10 connected to the shaft in a distal portion of the shaft; an inflation port 21 at the hub in fluid communication with the balloon. The shaft further has a guidewire lumen 22 extending through the hub, the shaft, and the balloon hosting a preloaded guide wire 80. The balloon comprises a first distal portion 11, a second central portion 12 or also called second middle portion and a third proximal portion 13, wherein in its delivery configuration the first distal portion 11 of the balloon is bent down to the third proximal portion 13 of the balloon such that the first distal portion and the third proximal portion of the balloon are arranged adjacent to each other. In Fig. 2 the first distal portion 11 and the third proximal portion 13 of the balloon are shown to be arranged in a parallel configuration. Though, it is readily understood by the skilled person that the first and the third balloon may also be arranged partially parallel to each other, where a distal portion of the third proximal portion and a proximal portion of the first distal portion are arranged in parallel next to each other while a distal portion of the first distal portion of the balloon and a proximal portion of the third proximal portion of the balloon are arranged to each other at an angle to each other.

As depicted in Fig. 2, in the delivery configuration of the catheter, where the first distal portion 11 of the balloon is bent down or folded down to the third proximal portion 13 of the balloon, the second central portion 12 of the balloon serves as bending position of the balloon. The central portion or middle portion of the balloon allows the balloon to bend by 180° without kinking or blocking the guidewire lumen and without blocking the balloon lumen.

Generally, a variety of dilatation balloon catheters, are known and suitable for the stent delivery system of the invention. The elongated catheter is sized and configured for delivery through a tortuous anatomy. For purpose of illustration only, the catheter shaft 2 embodied herein comprises an outer tubular member 24 and an inner tubular member 25. As shown in Fig. 2, the inner tubular member 25 defines the guidewire lumen 22 for a preloaded guidewire 80. Although Fig. 2 illustrate the guidewire lumen 22 with an over-the-wire (OTW) configuration, the guidewire lumen 22 can be configured for rapid-exchange (RX) delivery, as is well known in the art. Alternatively, the catheter body can include a fixed guidewire to permit the catheter to be delivered to a vessel location without the use of a separate guidewire. The fixed guide wire extends beyond the distal end of the balloon. Optionally, the distal end of the guide wire may be shaped as a hook.

Between the outer tubular member 24 and the inner tubular member 25 an inflation lumen 30 extending between the proximal end portion and the distal end portion of the catheter shaft 2 is defined. Specifically, as illustrated in Fig. 2, the coaxial relationship between the inner tubular member 25 and the outer tubular member 24 defines an annular inflation lumen 30 therebetween. The balloon 10 is provided in fluid communication with the inflation lumen 30. The inflation lumen 30 can supply fluid under pressure to expand the balloon 10 to a deployed condition, and if desired establish negative pressure to deflate the balloon 10. The expandable balloon 10 can thus be inflated and deflated using the inflation lumen 30. Suitable materials and techniques are well known for construction of the catheter shaft.

As depicted in Fig. 2 the single balloon 10 of the balloon catheter of the present invention may comprise at least a first waist portion 101 between the first distal portion 11 and the second central portion 12 and at least a second waist portion 102 between the second central portion 12 and the third proximal balloon portion 13.

A waist portion is defined as an area of the balloon having a reduced outer diameter compared to the adjacent balloon portions. In one embodiment the waist portion has a diameter which is reduced by at least 50% compared to the adjacent balloon portions. The length of the waist portion is short such that the waist portion constitutes a constriction or local narrowing. In one embodiment the length of the waist is up to 4mm, preferably up to 2 mm, preferably up to 1 mm. It is explicitly stated that the balloon wall is not attached to the catheter shaft in the area of the waist portion. That means, that the balloon has one single lumen and one single in- or deflation lumen, i.e. the balloon segments share one single lumen, namely the balloon lumen. The purpose of waist portions 101; 102 is to increase the bending flexibility of balloon when inflated. The middle portion of the balloon allows the balloon to bend by 180° without kinking or blocking the balloon lumen. Also, with the middle portion of the balloon acting as a flexural element the balloon according to the invention will keep its bent down delivery configuration during inflation. Even high pressure in the balloon lumen do not force the balloon to unbend into its retraction configuration or to reduce the angle at which the first balloon portion is bent down onto the third balloon portion. Further the second balloon portion or flexural element of the balloon catheter provides high torqueability to the catheter thus allowing the physician to rotate the catheter within the body vessels in order to facilitate placement of the distal portion of the balloon in the contra-lateral branch correctly.

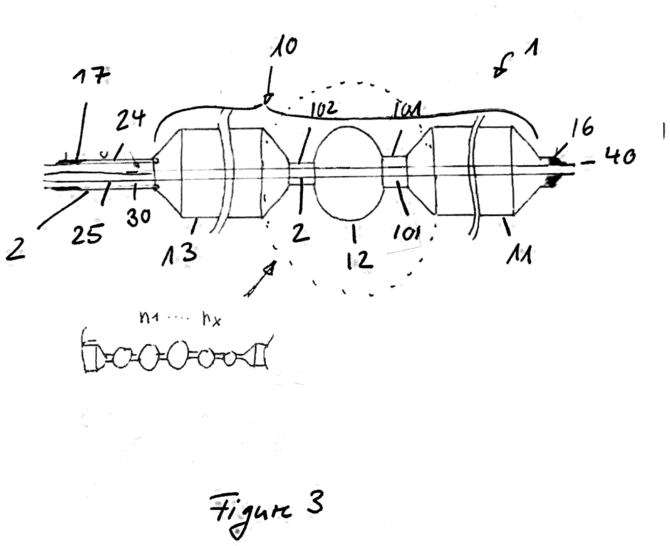

Whereas Fig. 2 shows the balloon catheter of the present invention in a bent down configuration which is the configuration in which the balloon is intended to be delivered to the treatment site as well as the configuration the balloon is inflated for treatment of a lesion in the bifurcated blood vessel, Fig. 3 depicts one embodiment of the balloon catheter 1 in its retracted configuration where the single balloon 10 is shown to be substantially straight to show the construction principle. The balloon catheter will assume this configuration when removed from the bifurcated blood vessel upon deflation.

As can be seen in Fig. 3 in further detail in one embodiment of the present application the balloon catheter comprises a catheter shaft 2; and a single balloon 10 connected to the shaft in a distal portion of the catheter. The catheter shaft 2 comprises an outer tubular member 24 and an inner tubular member 25. The inner tubular member 25 defines the guidewire lumen 22 for a guidewire (not shown). Between the outer tubular member 24 and the inner tubular member 25 an inflation lumen 30 extends between the proximal end portion and a distal end portion of the catheter shaft 2. A distal end of the balloon 16 is fluid-tightly attached to the distal end of the inner tubular member 25 to form a catheter tip 40. A proximal end of the balloon 17 is fluid-tightly attached to the distal end of the outer tubular member 24.

As illustrated in Figs. 2 and 3, the coaxial relationship between the inner tubular member 25 and the outer tubular member 24 defines an annular inflation lumen 30 therebetween. The balloon 10 is thus provided in fluid communication with the inflation lumen 30. The balloon 10 of the balloon catheter depicted in Fig.3 comprises a first waist portion 101 between the first distal balloon portion 11 and the second central balloon portion 12 and a second waist portion 102 between the second central balloon portion 12 and the third proximal balloon portion 13.

A can be seen in Fig 3 the waist portions 101, 102 are areas of reduced balloon diameter. The reduction in diameter is at least 50%. The balloon is only attached to the catheter shaft at its distal 16 and proximal 17 end. The balloon is not attached to the catheter shaft (or inner or outer tubular member) at its waist portions.

This configuration of the second middle portion 12 of the balloon being bordered by at least one waist portion 101 distal of the second middle balloon portion and at least one waist portion 102 proximal of the second middle balloon portion allows for bending the balloon or folding down the balloon by 180° at its middle portion without stretching the balloon and without constriction of the inflation lumen. The single balloon can thus be easily and quickly inflated and deflated.

As mentioned before, the individual balloon portions may be of rotational symmetric shape, cylindrical shape, conical shape, stepped shape or of a tapered shape. For the central balloon portion 12 a spherical- like shape is preferred. In some further embodiments the central balloon portion 12 may comprise one or more additional waist portions, i.e. portions of reduced outer diameter compared to the adjacent balloon portions. As depicted in Fig. 3 for sake of example but not limiting thereto, the central balloon portion may be configured of a series of shorter balloon portions intermitted by one, 2, 3, 4, 5, 6, 7, 8, 9, 10 or more additional waist portions. The shorter balloon portions may be spherical-like shaped or assume any other suitable shape to allow for unconstricting bending or folding down of the balloon.

Upon placement of a bifurcated stent in a bifurcated vessel it is often use, independent of the system or technique applied to, to post-dilatate a proximal portion of the main stent or aortic stent employing a balloon having a greater diameter. This is done to ensure improved contact of the main stent portion to the vessel wall in order to ensure a smooth transition and to avoid turbulences, blood clotting and restenosis in this area. This procedure requires an additional balloon.

In order to address this need, a further embodiment of the present invention comprises a balloon catheter according to the present invention as described in detail above, the balloon catheter further comprising a second additional balloon located proximal of the first balloon and mounted on the catheter shaft. The second balloon is in fluid communication with a second inflation lumen and a second inflation port at the hub at the proximal end of the shaft.

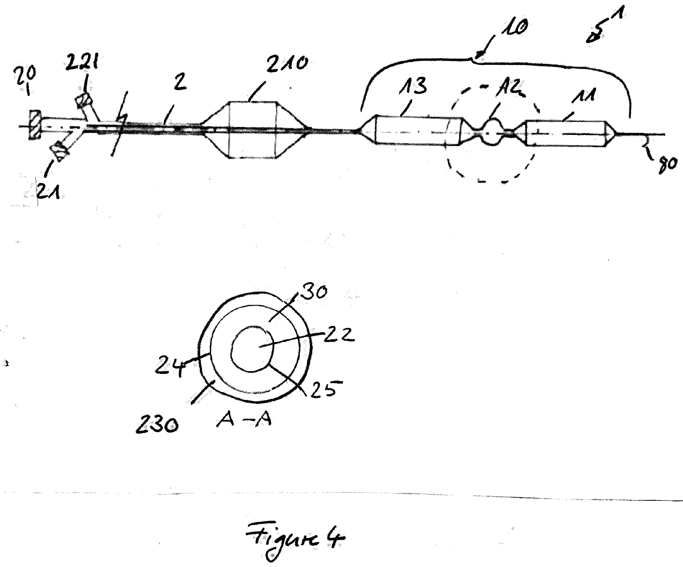

As shown in Fig. 4 in one embodiment the balloon catheter 1 for delivering a stent into a bifurcated blood vessel, comprises a proximal hub 20; a shaft 2; and a single balloon 10 connected to the shaft in a distal portion of the shaft; a first inflation port 21 at the hub in fluid communication with the balloon. The shaft further has a guidewire lumen 22 hosting a preloaded guide wire 80 and extending through the hub, the shaft, and the balloons 10, 210. The single balloon comprises a first distal balloon portion 11, a second central balloon portion 12 and a third proximal balloon portion 13, wherein in its delivery configuration the first distal portion 11 of the balloon is bent down to the third proximal portion 13 of the balloon 10 such that the first distal portion and the third proximal portion of the balloon are arranged adjacent to each other (Fig. 4 depicts the catheter in its unbent configuration). The catheter further comprises one further balloon 210 located proximal of the first single balloon 10 and mounted on the catheter shaft. The second balloon 210 is in fluid communication with a second inflation lumen 230 and a second inflation port 221 at the hub at the proximal end of the shaft.

The second balloon 210 has an outer diameter that is larger than a maximum diameter of the single balloon 10. In another embodiment, the outer diameter of the second additional balloon is equal or greater than the outer diameter of the first distal portion and the third proximal portion of the balloon in the bent down configuration.

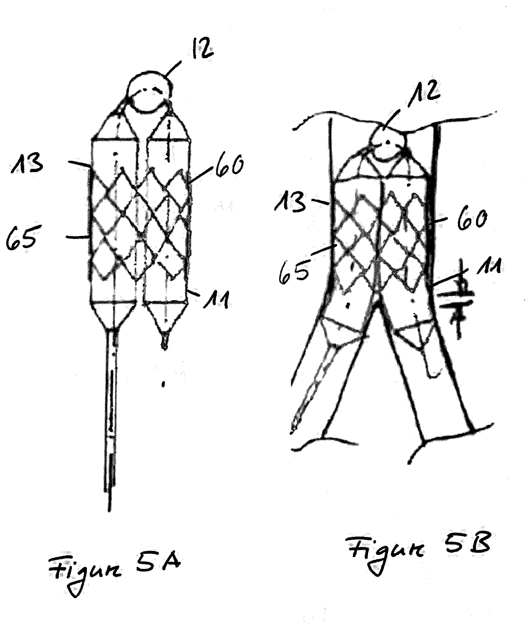

Referring now to FIGs 5A and B illustrating a medical device according to an embodiment of the present application. The medical device comprises the catheter of the present invention and a stent mounted thereon.

Fig. 5A depicts a medical device system for treating a lesion in a bifurcated blood vessel, the system comprising a stent 60 constituted of a main body 65 and the catheter of the present invention for delivering the stent at the bifurcated blood vessel.

The catheter comprises a single balloon having a first distal portion 11, a central portion 12 and a third proximal portion 13, wherein a main body of a stent 65 is crimped onto both, a proximal portion of the first distal portion and onto a distal portion of the third balloon portion, wherein the first balloon portion 11 is bent down onto the third balloon portion 13. The first distal balloon portion and the third proximal balloon portion are thus arranged side-by-side to each other and the main portion of the stent is crimped on both, the first and the third balloon portion.

As can be seen in Fig. 5B illustrating the medical device of Fig. 5A being placed and expanded close to the bifurcation in a bifurcated vessel. Employing the folded down or bent-over balloon in a retrograde delivering approach allows for proper placement of the main stent portion in the main vessel but close to the bifurcation without the risk over over-dilatation of the branches. Accordingly, the balloon catheter of the present invention allows for exact, precise and non-harming delivery of a stent in the main artery in close proximity to the branches of the bifurcated vessel.

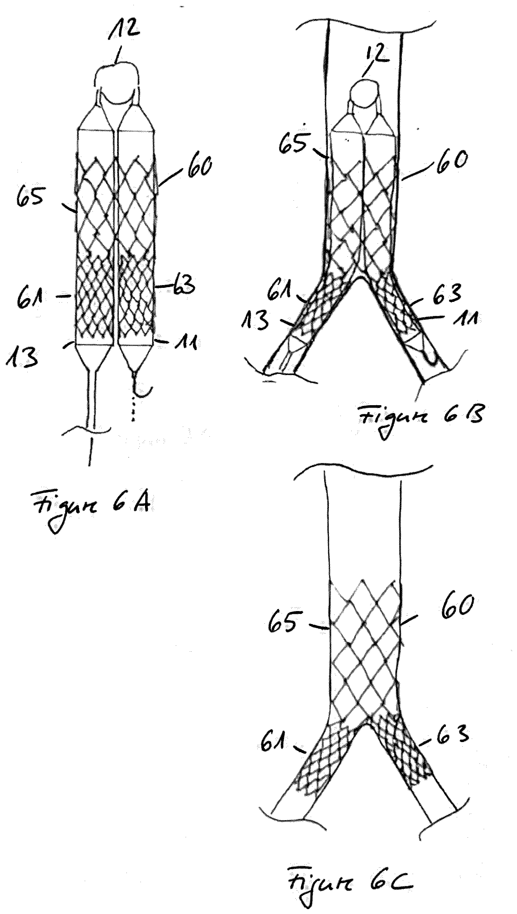

In other preferred embodiments of the present invention the stent 60 is a bifurcated stent comprising a main body stent 65, a first branch stent 63, and a second branch stent 61 which is mounted on a balloon catheter of the present invention for delivery into a bifurcated vessel. Fig. 6A depicts a medical device for treating a lesion in a bifurcated blood vessel, the system comprising a bifurcated stent comprising a main body stent portion, a first branch stent portion 63, and a second branch stent portion 61 ; a catheter for delivering the bifurcated stent into the bifurcated blood vessel, the catheter comprising a single balloon having a first distal portion, a second central portion and a third proximal portion; wherein the first branch stent 63 is crimped onto a distal portion of the first distal portion 11, the second branch stent 61 is crimped onto a proximal portion of the third proximal portion 13, and the main body stent 65 is crimped onto a proximal portion of the first distal portion and onto a distal portion of the third proximal portion, wherein the first balloon portion is bent down onto the third balloon portion.

As illustrated in Fig 6B the medical device is delivered into the bifurcation of a vessel through one ipsi-lateral access. Once the balloon catheter is deflated and removed, the bifurcated stent fully covers the bifurcation of the treated vessel (see Fig. 6C).

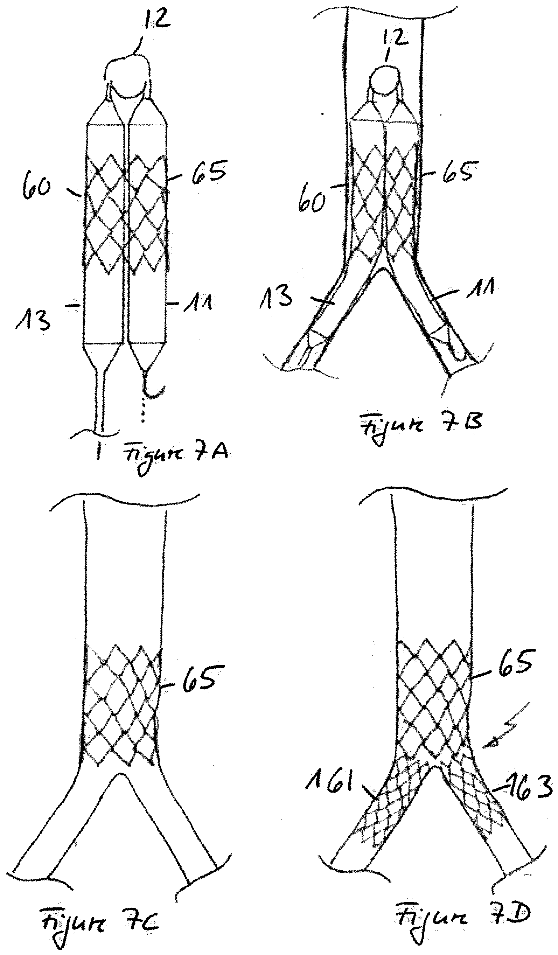

Another device and approach to treat a lesion in a bifurcated vessel is depicted in Figs. 7A to D. The device depicted in Fig. 7A corresponds to the device shown and described in Fig. 5. A main stent 65 is placed in proximity to the bifurcation employing a balloon catheter of the present invention and using one ipsi-lateral access site. In order to further cover the legs of the bifurcation a first leg stent 161 is employed into the first side branch using the ipsi-lateral access and a second leg stent 163 is employed into the second side branch using a contra-lateral access site. The leg stents may be placed adjacent to the main stent or may be placed in an overlapping configuration with a proximal portion of the main stent.

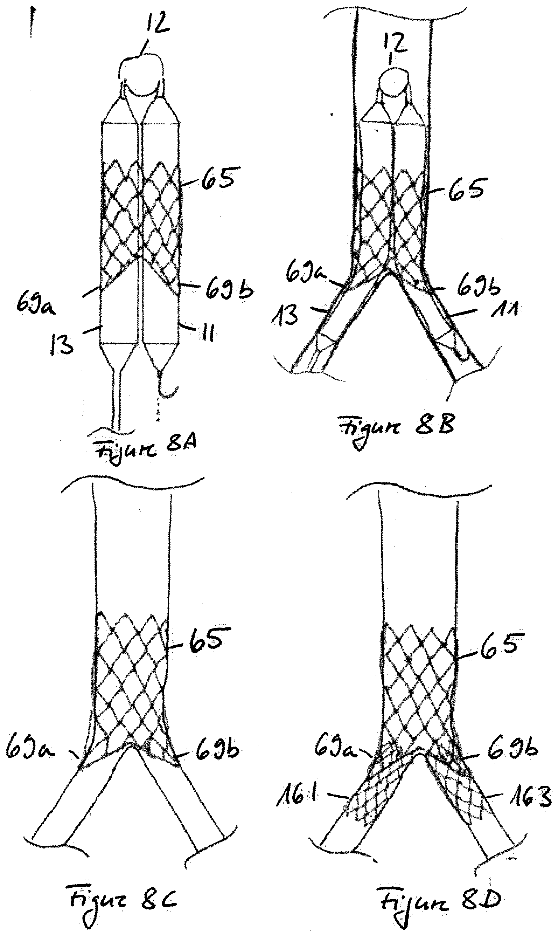

A similar approach is depicted in Figs. 8A to D. The main stent in this embodiment differs from the main stent as shown in Figs. 7A to D in that the main stent 65 of this alternative embodiment comprises two wing-like structures 69a, 69b at its proximal end. Each of these wings reaches in one of the side branches of the bifurcation thus increasing the wall coverage and support in the transition area between the main body stent 65 and the side branches. In case side branch stents 161, 163 are employed in two subsequent delivery steps in the same manner as described for the embodiment of FIGs 7 A to D, the wings overlap with the distal portions of the side branch stents and thus increase the wall coverage and support in the transition area between the main body stent 65 and the side branch stents 161, 163.

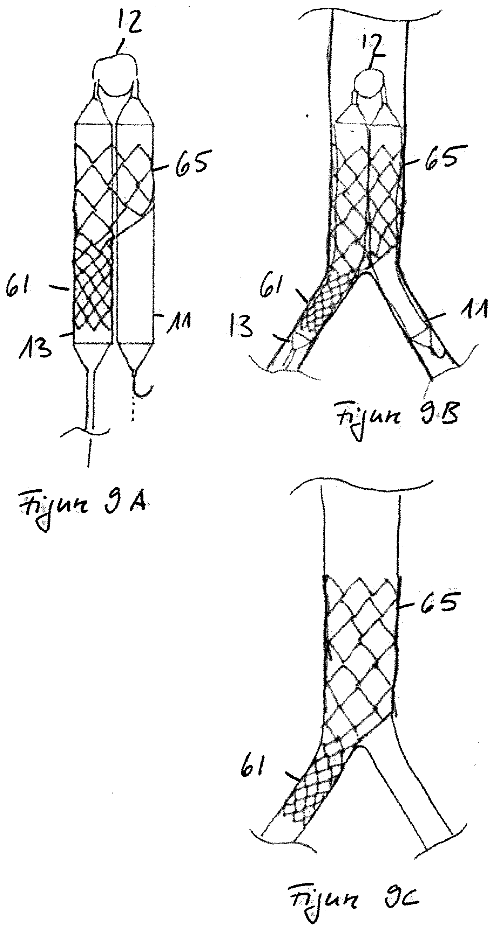

In other embodiments the bifurcated stent mounted on the balloon catheter of the present invention comprises a main body stent 65 and one branch stent 61 formed integrally, i.e. in one piece. As shown in Figs. 9A to C, a stent comprising a main body stent 65 and one branch stent are loaded onto the balloon catheter by mounting the main body of a stent 65 onto both, a proximal portion of the first distal portion 11 and onto a distal portion of the third balloon portion 13, wherein the first balloon portion is bent down onto the third balloon portion, and wherein the one branch stent is crimped onto a proximal portion of the third proximal portion of the balloon such that the side branch stent covers the branch of the bifurcation that has been used as an access site for the catheter, i.e. the ipsi-lateral vessel.

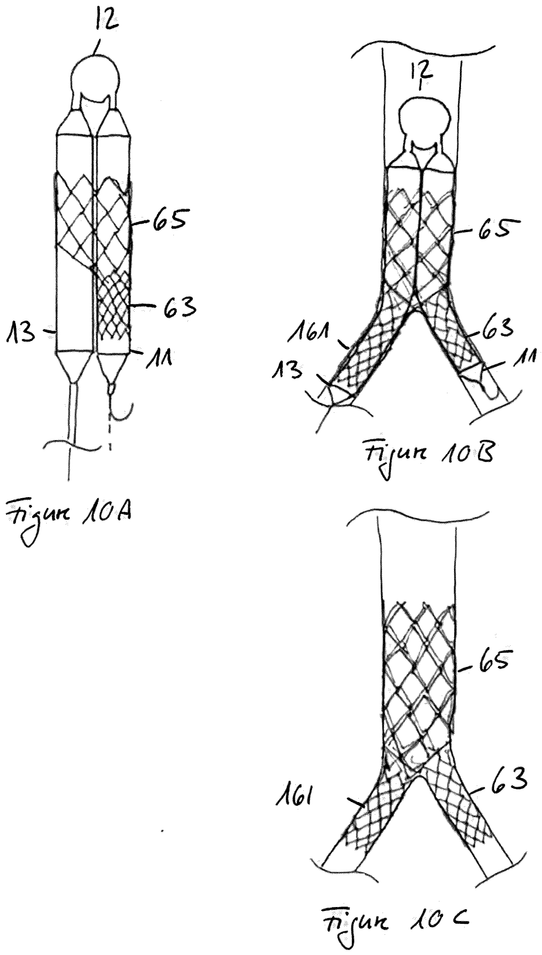

As shown in Figs. 10A to C, a stent comprising a main body stent 65 and one branch stent 63 are loaded onto the balloon catheter by mounting the main body of a stent 65 onto both, a proximal portion of the first distal portion 11 and onto a distal portion of the third balloon portion 13, wherein the first balloon portion is bent down onto the third balloon portion, and wherein the one branch stent 63 is crimped onto a distal portion of the first distal portion of the balloon such that the side branch 63 stent covers the contralateral branch vessel of the bifurcation i.e. the vessel. In a second step, a second branch stent 161 can then be deployed into the ipsi-lateral vessel by using the same access site in the procedure.

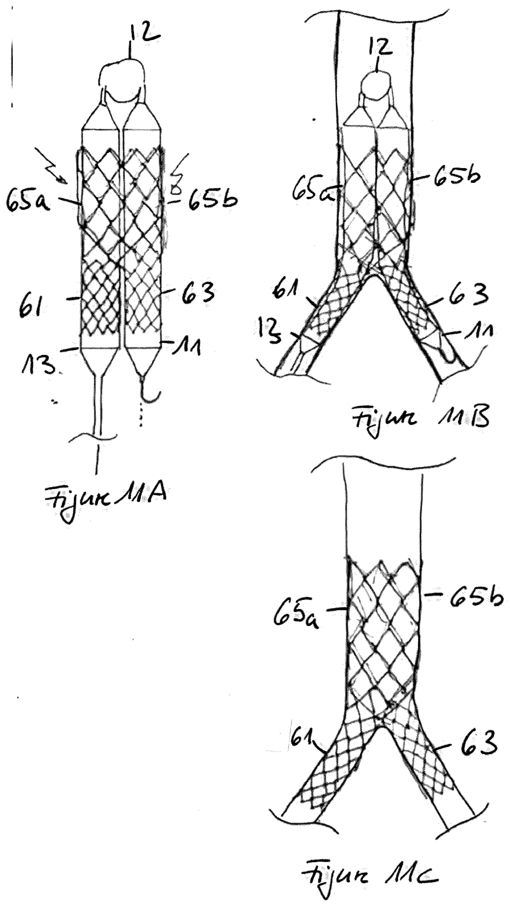

In a further embodiment of the present invention (Figs. 11A to C) , a first stent comprising a main body stent 65a and a first branch stent 63 and a second stent comprising a main body stent 65b and a second branch stent 61 are loaded onto the balloon catheter by mounting the main bodies 65a, 65b of the first and the second stent in an overlapping manner onto both, a proximal portion of the first distal portion 11 and onto a distal portion of the third balloon portion 13, wherein the first balloon portion is bent down onto the third balloon portion, and wherein the first branch stent 63 of the first stent is crimped onto a distal portion of the first distal portion 11 of the balloon, and wherein the second branch stent 61 of the second stent is crimped onto a proximal portion of the third proximal portion 13 of the balloon.

While the above is a complete description of the preferred embodiments of the invention, various alternatives, modifications, and equivalents may be used. The various features of the embodiments disclosed herein may be combined or substituted with one another. Therefore, the above description should not be taken as limiting in scope of the invention which is defined by the appended claims. Moreover, any combination of the above-described elements in all possible variations thereof is encompassed by the invention unless otherwise indicated herein or otherwise clearly contradicted by context. It is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein.

## Claims

1. A balloon catheter for delivering a bifurcated stent into a bifurcated blood vessel, the balloon catheter comprising: a hub; a shaft; and a single balloon connected to the shaft; an inflation port at the hub in fluid communication with the single balloon; wherein the single balloon comprises a first distal portion, a second central portion and a third proximal portion, wherein in its delivery configuration the first distal portion of the balloon is bent down to the third proximal portion of the balloon such that the first distal portion and the third proximal portion of the balloon are arranged adjacent to each other.

2. The balloon catheter of claim 1, wherein the balloon catheter comprises a guidewire lumen hosting a preloaded guide wire and extending through the hub, the shaft, and the balloon.

3. The balloon catheter of claim 1, wherein the balloon catheter comprises a fixed guide wire extending beyond the distal end of the balloon.

4. The balloon catheter according to any one of claims 1 to 3, wherein the single balloon comprises at least a first waist portion between the first distal portion and the second central portion and at least a second waist portion between the second central portion the third proximal balloon portion.

5. The balloon catheter according to any one of claims 1 to 4, wherein the first distal portion and the third proximal portion of the single balloon are of rotational symmetric shape, and/or cylindrical shape, and/or conical shape, and/or stepped shape cylindrical in shape.

6. The balloon catheter according to any one of claims 1 to 5, wherein the second central balloon portion of the single balloon is spherical-like in shape.

7. The balloon catheter according to any one of claims 1 to 5, wherein the second central balloon portion of the single balloon is formed from a series of spherical-like shaped balloon portions intermitted by 1 to 10 waist portions.

8. The balloon catheter according to any one of claims 1 to 7, wherein the single balloon maintains its bent down configuration when inflated in the bifurcated blood vessel.

9. The balloon catheter according to any one of claims 1 to 8, wherein the balloon catheter comprises a second balloon mounted to the catheter shaft proximal of the single balloon and being in fluid communication with a second inflation lumen.

10. The balloon catheter of claim 9, wherein the second balloon has an outer diameter that is larger than a maximum diameter of the single balloon.

11. The balloon catheter of any one of claims 1 to 10, with a stent being mounted onto the single balloon, wherein a main body of a stent is mounted onto both, a proximal portion of the first distal portion and onto a distal portion of the third balloon portion, while the first balloon portion is bent down onto the third balloon portion.

12. The balloon catheter of any one of claims 1 to 10, with a bifurcated stent comprising a main body stent, a first branch stent, and a second branch stent being mounted onto the single balloon, wherein the first branch stent is mounted onto a distal portion of the first distal portion, the second branch stent is mounted onto a proximal portion of the third proximal portion, and wherein the main body of the stent is mounted onto both, a proximal portion of the first distal portion and a distal portion of the third balloon portion, while the first balloon portion is bent down onto the third balloon portion.

13. The balloon catheter according to claim 12, wherein the main body stent portion, the first branch stent portion, and the second branch stent portion of the stent are formed in one piece or wherein the main body stent portion, the first branch stent portion, and the second branch stent portion of the stent are formed each by one single stent.

14. The balloon catheter according to any one of the preceding claims, wherein the stent is a stent graft.

15. A method for placing a stent into a vessel in immediate proximity to a bifurcation in a vessel of a patient comprises:
a) mounting a stent including a main body stent onto a catheter having a single balloon having a first distal portion, a second central portion and a third proximal portion; wherein the main body stent is crimped onto a proximal portion of the first distal portion and onto a distal portion of the third proximal balloon portion, wherein the first balloon portion is bent down on the third balloon portion;
b) advancing the catheter with the stent mounted onto the balloons from an ipsi-lateral side vessel past the bifurcation;
c) placing the stent in the main vessel of a patient in immediate proximity to a bifurcation of the vessel into the bifurcating blood vessel by partially retracting the catheter;
d) expanding the main body stent into the main vessel by inflating the balloon;
e) deflating the balloon;
f) retracting the catheter through the ipsi-lateral side vessel, and
g) removing the catheter from the patient.
